# EUROPÄISCHE PATENTSCHRIFT

(11) **EP 0 930 064 B1**
(45) Veröffentlichungstag und Bekanntmachung des Hinweises auf die Patenterteilung: **02.05.2002**
(21) Anmeldenummer: 98123261.4
(22) Anmeldetag: 07.12.1998
(51) Int. Cl.: A61K 7/48, A61K 7/50, A61K 7/00

(54) **Balneologische Badezusätze, insbesondere in Form einer Brausetablette, Verfahren zu ihrer Herstellung und ihre Verwendung**
Bath additive for balneotherapy in the form of an effervescent tablet, process for preparing the same and use thereof
Additif pour le bain sous la forme de comprimé effervescent à utiliser dans la balneothérapie, son procedé de préparation et son utilisation

(30) Priorität: 20.12.1997 DE 19757059
(43) Veröffentlichungstag der Anmeldung: 21.07.1999
(73) Patentinhaber: Merz & Co. GmbH & Co., D-60318 Frankfurt (DE)
(72) Erfinder: Nürnberg, Eberhard, Prof.Dr., 91080 Uttenreuth (DE); Jerzembek, Kerstin, 64823 Gross-Umstadt (DE); Beutler, Rolf D., Dr., 64739 Höchst/Hummetroth (DE); Ebinger, Jürgen, 65510 Hünstetten 8 (DE); Weis, Ruth, 64331 Weiterstadt (DE)
(74) Vertreter: Beil, Hans Christoph, Dr.

(56) Entgegenhaltungen:
- EP-A- 0 312 668
- EP-A- 0 498 272
- EP-A- 0 557 825

## Beschreibung

Die vorliegende Erfindung betrifft einen neuen Typ von festen balneologischen Zubereitungen zur kosmetischen, hygienischen und therapeutischen Anwendung, der sowohl die Eigenschaften eines festen Badezusatzes als auch die von flüssigen Produkten aufweist und insbesondere durch Zusatz von Lipidkomponenten, vesikelbildenden Lipiden, Tensiden und ggf. Mineralsalzen gekennzeichnet ist. Auch können wirksame Komponenten, Hilfsstoffe, wie Stabilisatoren, adsorbierende Stoffe, Schmiermittel sowie Gleit- und zerfallsfördernde Mittel enthalten sein..

Die Erfindung betrifft ferner die Herstellung solcher Produkte und ihre Anwendung.

Bisher wurden als balneologische Produkte vorwiegend flüssige Zubereitungen in Form von Tensidlösungen unter Zusatz von ätherischen Ölen sowie weiteren wirksamen Komponenten, Gelen, insbesondere für Duschzwecke oder in Form von gelösten Salzen angewendet. Unter den flüssigen Zubereitungen nehmen diejenigen Tensidprodukte, die einen Anteil - eventuell sehr hohen Anteil - an Ölen und anderen fettartigen Produkten enthalten, eine Sonderstellung ein.

Ein besonderer Vorzug dieser Ölbäder ist insbesondere dadurch gegeben, daß sie als Zusätze vesikelbildende Lipide enthalten und zur spontanen Liposomenbildung befähigt sind (DE-PS 42 05 548).

In diesen flüssigen Zubereitungen sind normalerweise keine O/W-Tenside enthalten, da sie eine spontane Vesikelbildung behindern können. Nur unter bestimmten Voraussetzungen gelingt es, Badepräparate mit einem Lipidanteil und O/W-Tensiden mit vesikelbildenen Eigenschaften darzustellen. Dies ist in der DE-OS 196 02 346.7-41 dargelegt. Danach können Zubereitungen mit einem Gehalt von mehr als 30 % an lipophilen Komponenten und vesikelbildenden Substanzen eine spontane Vesikelbildung ergeben, wenn eine Komplexierung des O/W-Tensids mit W/O-Tensiden vorgenommen wird. Das molare Verhältnis von W/O- zu O/W-Tensiden beträgt 1:2 bis 1:0,2.

Die obengenannten Oleobalneologika stellen entweder flüssige oder halbfeste, streichfähige Präparate dar. Sie enthalten jedoch keine Elektrolyte, da diese die Konsistenz der Zusammensetzung in unerwünschter Weise beeinflussen können. Darüberhinaus sind hinsichtlich des Transports und der Dosierung solcher Produkte besondere Maßnahmen zu ergreifen.

Feste balneologische Produkte, wie z.B. Badetabletten, die sich durch die Freisetzung von CO₂ in der Badeflotte infolge ihres Brausecharakters auszeichnen, sind von verpackungstechnischen - insbesondere bei Glasflaschen (Verletzungsgefahr) - und transporttechnischen Gesichtspunkten und der Dosierung einfacher zu handhaben und können auch Elektrolyte aufweisen. Sie sind zusammengesetzt aus einem sogenannten Brausesatz aus Carbonat/Bicarbonat und einer Säure, vorzugsweise Zitronen- oder Weinsäure, Bindemitteln (Cellulosederivate, Stärke), Spreng- und Schmiermitteln (Talkum, Macrogol (Polyethylenglykol)) sowie ätherischen Öl-Zusätzen, Parfümstoffen, Farbstoffen und Tensiden.

Kohlensäure vermittelt nicht nur ein angenehm prickelndes Badegefühl. Sie wirkt auch gefäßerweiternd auf die Hautgefäße, durchblutungssteigemd und regulierend auf die Wärmeund Kälterezeptoren der Haut, d.h. auf die Sinnesorgane, die das Empfinden von Wärme und Kälte vermitteln.

Solche sprudelnden Badesalztabletten sind beispielsweise in G.A. Nowak, "Die kosmetischen Präparate", 2. Auflage, 1975, S. 672-674 beschrieben und umfassen z.B. Zusammensetzungen aus Natriumbicarbonat, Weinsäure, Talkum, Natriumhexametaphosphat, Kartoffelstärke, Carboxymethylcellulose, Pektin, Natriumlaurylsulfat, ggf. Farbstoffe und Parfümöl.

In K.H. Schrader, "Grundlagen und Rezepturen der Kosmetika", 2. Auflage 1989, S. 606 wird ein nicht sprudelndes Badesalz aus Natriumchlorid und Natriumsulfat bzw. auf S. 390 der

Ausgabe 1979 sprudelnde Badetabletten aus Natriumhydrogencarbonat, Wein- und Borsäure, Weizenstärkepulver, Kaolin, Parfüm und Apfelpektin beschrieben.

EP-A-0 498 272 offenbart einen sprudelnden Badezusatz, der eine Mischung von Carbonatsalz und Säure, sowie 0,1 bis 50 Gew.-% (auf dem Gesamtgewicht) eines Kolloidmaterials enthält. Es ist vorteilhaft ein nonionisches Tensid mit HLB 7 oder höher einzusetzen. Das Kolloidmaterial ist bevorzugt Hafermehl in einer Menge von 1-30 Gew.-% (auf dem Gesamtgewicht).

Wie hieraus ersichtlich ist, enthalten - insbesondere sprudelnde - Badetabletten keine Tensid-Lipid-Kombination mit hautreinigender, pflegender Wirkung.

Will man die aus flüssigen Badepräparaten bekannten Erkenntnisse bezüglich der Tensid-Lipid-Kombination auf feste Produkte wie sprudelnde Badetabletten übertragen, so gelingt dies nicht, da Komprimate nur äußerst geringe Flüssigkeits- und Fettanteile enthalten können, um stabil zu bleiben.

Ein Zusatz von flüssigen oder pastösen Lipiden und Tensiden beeinflußt nämlich die Fließfähigkeit, Tablettierbarkeit und die galenisch relevanten Eigenschaften der Komprimate hinsichtlich Friabilität, Festigkeit und Auflösungseigenschaften nachteilig bzw. macht die Gewinnung einzeln dosierter fester (Arznei)Formkörper unmöglich.

Die Aufgabe vorliegender Erfindung ist es daher, an sich bekannte Badezusätze, insbesondere Brausetabletten so zuzubereiten, daß sie sowohl die Eigenschaften fester sprudelnder Tabletten als auch die Eigenschaften flüssiger Bäder/Ölbäder aufweisen, also Lipide, vesikelbildende Zusätze, die eine spontane Vesikelbildung beim Auflösen in Wasser ermöglichen, sowie Tenside mit O/W- und/oder W/O-Emulgatorcharakter und ggf. auch weitere Elektrolyten außer denjenigen, die als Brausesatzkomponenten eingesetzt werden, enthalten.

Dadurch sollen insbesondere feste Badetabletten mit günstigen Auflösungseigenschaften in Wasser unter gleichzeitiger Emulgierung der Lipidkomponente(n) und ggf. Freisetzung von Elektrolyten nach dem Vorbild der sogenannten Akratothermen sowie eine spontane Vesikelbildung unter Bildung eines hautpflegenden, feinporigen Schaums hergestellt werden, so daß sowohl die Eigenschaften fester als auch flüssiger Produkte vereint werden können.

Bei der Lösung dieser Aufgabe konnten die in den genannten Patenten bzw. Patentanmeldungen und Literatur gewonnenen Präparate bezüglich der Herstellung von lipophilen vesikelbildenden Badepräparaten nicht verwendet werden. Derartige Produkte können nicht in einer Weise, wie es für die angestrebten balneologischen Brausetabletten erforderlich wäre, inkorporiert werden.

Überraschenderweise wurde nun aber gefunden, daß stabile Badezusätze, insbesondere feste sprudelnde Badesalztabletten hergestellt werden können, wenn man dem festen Brausesatz aus Carbonat/Bicarbonat und Säure bestimmte Anteile einer oder mehrerer Lipidkomponenten, eines vesikelbildenden Lipids in Verbindung mit W/O- und/oder O/W-Tensiden zusetzt. Insbesondere bei weiterem Zusatz von Mineralsalzen können thermalbadanaloge Salzmilieus zusammen mit balneologischen Effekten von Reinigungs-, Heil- und Pflegebädern erzielt werden.

Erfindungsgemäße Badezusätze in Form von Brausetabletten umfassen 10-97 %, insbesondere 60-92%, einer Carbonat-/Bicarbonat-Säure-Mischung, 0-30 %, insbesondere 0-10% wirksame Komponenten, ausgewählt aus ätherischen Ölen, Parfümstoffen, Vitaminen, Extrakten, kurativen oder hautpflegenden Substanzen oder deren Mischungen, 0-10 %, Hilfsstoffe, und sind dadurch gekennzeichnet, daß sie weiterhin
0,1-50 %, insbesondere 1-25%, einer oder mehrerer Lipidkomponente;
0,1-20 %, insbesondere 0,1-5%, vesikelbildende Lipide;
0,1-20%, insbesondere 0,1-5%, eines oder mehrerer O/W- und/oder W/O-Tensid(e)s;
0-10 %, insbesondere 0,1-3%, eines Kolloids mit einer Molekülmasse bis maximal 50 000;
sowie
0-80% Mineralsalze
aufweisen.

Bevorzugte Tabletten umfassen Kombinationen eines oder mehrerer O/W und W/O Tenside oder eines oder mehrerer O/W Tenside in den angegebenen Mengen Bevorzugte erfindungsgemäße Brausetabletten enthalten 60-92 % der Carbonat/Säure-Mischung; 0,1 - 5 % Tenside, 1 - 25 % Lipidkomponente; 0,1 - 5 % vesikelbildende Lipide; 0 - 5 % adsorbierende Stoffe; 0 - 10 % wirksame Komponenten; 0,1 - 3 % Eiweißhydrolysat und 0 - 80 % Mineralsalz.

Besonders bevorzugte Brausetabletten umfassen
70 - 92 % der Carbonat/Bicarbonatmischung,
0,2 - 5 % wirksame Komponenten, insbesondere Alpenkräuterextrakt/Kombination aus Campher, Eucalyptusöl, Teebaumöl oder Weidenrindenextrakt/Methylnicotinat oder Grüner-Tee-Extrakt/Rosmarinöl,
0-5 % Hilfsstoffe, wie insbesondere Farb- und Parfümstoffe;
1-10 % eines oder mehrerer Lipide;
0,1-5 %, insbesondere 1-4 %, Tenside;
0,1-5 % vesikelbildende Lipide;
0,5-2,5 % eines Kolloids, insbesondere Eiweißhydrolysat, ausgewählt aus Weizenprotein und/oder Collagenhydrolysat,
sowie 4 - 55 % Minalsalze, insbesondere solche von Calcium, Magnesium, Natrium und Kalium.

Die Carbonat-/Bicarbonat-Säuremischung ist bekannt und besteht üblicherweise aus 5-80% Carbonat/Bicarbonat, vorzugsweise 10-50%, wie z.B. Natrium- oder Kalium(bi)carbonat oder Ammonium(bi)carbonat, Kaliumcarbonat sowie 10-80 %, vorzugsweise 10-50 %, einer Säure, insbesondere einer organischen Säure, wie z.B. Zitronen-oder Weinsäure oder Fumarsäure oder Adipinsäure oder deren Mischungen, oder eines Säureanhydrids wie Zitronen- oder Bernsteinsäureanhydrid; oder anorganischen sauren Salzen, wie Natriumdihydrogenphosphat, Natriumfumarat oder deren Mischungen.

Die erfindungsgemäßen Zusammensetzungen können, wie erwähnt, 0-30 % wirksame Komponenten oder Mischungen hiervon enthalten.
Hierzu gehören ätherische Öle, wie z.B. Rosmarinöl, Eucalyptusöl, Teebaumöl, oder Parfümstoffe, wie sie für solche Zwecke allgemein eingesetzt werden, oder Vitamine, wie z.B. Vitamin E, Pantothensäure, Vitamin C, und kurative Stoffe mit einer kutanen und/oder systemischen Wirkung, wie beispielsweise Polidocanol, Methylnicotinat, Hydroxyethylsalicylat, Harnstoff. Ferner können auch hautpflegende Komponenten, wie Jojobaöl, Avocadoöl, eingesetzt werden. Je nach Anwendungszweck können die wirksamen Komponenten auch kombiniert werden.

Als Hilfsstoffe werden, falls erforderlich, adsorbierende Stoffe, Gleitmittel, Sprengmittel, Farbstoffe oder sonstige für Brausetabletten übliche Substanzen in den angegebenen Mengen eingesetzt.

Als adsorbierende Stoffe eignen sich insbesondere Cyclodextrin, Dextrin, Stärke und/oder hochdisperse Kieselsäure oder andere Silicumdioxid-Produkte in den angegebenen Mengen.

Geeignete Farbstoffe sind beispielsweise wasserlösliche Farbstoffe, wie z.B. Cochenillerot, Patentblau und Chinolingelb.

Als Gleitmittel eignen sich insbesondere Macrogole,Magnesiumstearat und Talcum.

Als Sprengmittel können Stärkeprodukte, Cellulosederivate und carboxymethylierte, quervernetzte Povidone eingesetzt werden.

Als Mineralsalze werden insbesondere Salze aus den Kationen Natrium, Kalium, Magnesium, Calcium, Eisen, Ammonium oder Mangan und den Anionen Hydrogencarbonat, Chlorid, Fluorid, Sulfat oder Nitrat als solche oder als Mischungen eingesetzt. Ferner können auch andere aus dem Thermalbadmileu bekannte Salze, wie z.B. bei den Kationen Lithium, Aluminium, Strontium, und bei den Anionen lodid, Bromid, Thiosulfat, eingesetzt werden. Hierdurch können thermalbadanaloge Bedingungen erzielt werden.

Mit den obengenannten Mineralsalzen, insbesondere Stein- und/oder Meersalz, können erfindungsgemäße Zusammensetzungen auch als Granulat direkt eingesetzt werden.

Überraschenderweise zeigte sich weiterhin, daß trotz des Vorhandenseins der Elektrolyte (aus Brausesatz und ggf. den Mineralsalzen) weder die spontane Vesikelbildung noch die Hautreinigung/Pflege aufgrund der Tensid-Lipid-Mischung negativ beeinflußt wird.

Diese spontane Vesikelbildung war keinesfalls vorauszusehen, weil das primär eingesetzte vesikelbildende Lipid nicht wie bei den Ölbädern oder bei den Duscholeogelen in einem Öl gelöst, also molekulardispers verteilt vorliegt, sondern direkt in seiner eigentlichen Form (flüssig, pastös oder fest) dem Tablettiergut zugesetzt wird und darüberhinaus dieses vesikelbildende Lipid, weitere Lipide und die Tenside sowie sämtliche anderen Bestandteile - jeweils getrennt voneinander- in der Pulvermischung vorliegen.

Daß die Liposomenbildung auch nicht durch den hohen Elektrolytgehalt beeinflußt wird, war ebenfalls nicht vorauszusehen.
Darüberhinaus ist eine Direkttablettierung trotz des lipophilen Anteils möglich.

Ferner hat sich gezeigt, daß aufgrund der erfindungsgemäßen Kombination mechanisch stabile Tabletten mitunter auch ohne die üblichen Spreng- und Bindemittel hergestellt werden können. Diese zerfallen dennoch ausreichend schnell im Badewasser, sind besonders hautverträglich und schonend und verfügen über eine überraschend gute Lipidverteilung trotz des geringen Tensidanteils. Zudem tritt trotz des Vorhandenseins von O/W-Tensiden ggf. neben W/O-Tensiden eine spontane Vesikelbildung ein und angenehmer, feinporiger Schaum bildet sich aus.

Ggf. können Kolloide, insbesondere Eiweißhydrolysate pflanzlichen und/oder tierischen Ursprungs, wie z.B. Weizenproteinhydrolysate und Collagenhydrolysate, mit einer Molekülmasse kleiner/gleich 50 000 inkorporiert sein, die sich günstig auf die Liposomen-und Schaumbildung auswirken. Andere geeignete Kolloide sind z.B. Polyvinylpyrrolidon, Methylcellulose und Methylhydroxyethylcellulose. Sie werden in Mengen von 0-10%, inbesondere 0,1-3 %, eingesetzt.

Mit der erfindungsgemäßen Zusammensetzung können Tabletten mit einem hohen Gewicht, insbesondere 10-300 g, vorzugsweise 40-100 g, hergestellt werden, indem die festen Ausgangsmaterialien vermischt, (partial)granuliert und mit flüssigen Komponenten in geeigneter Weise vereinigt und verpreßt, oder nach dem Vermischen und Vereinigen sämtlicher Komponenten ohne Granulation direkt verpreßt werden.

Darüberhinaus kann die erfindungsgemäße Zusammensetzung nach dem Vermischen und Vereinigen sämtlicher Komponenten granuliert und direkt verwendet werden.

Besonders bevorzugte Lipid-Komponenten, wie sie in der erfindungsgemäßen Tablette eingesetzt werden können, sind z.B. hydriertes Sojaöl, hydriertes Rizinusöl, hydriertes Erdnußöl, Partialester oder Ester des Glyerols, des Sorbitols, des Ethylenglykols und des Propylenglykols sowie Mischester einschließlich der Sorbitanester sowie Wachse, Ester höherer Fettalkohole und/oder Fettsäuren oder Mischungen hiervon. Zu letzteren gehören beispielsweise Glycerolmonostearat, Propylenglykolmonostearat, Ethylenglykolmonostearat, Cetylpalmitat oder Partialester des Sorbitans oder Mischungen hiervon.

Besonders überraschend ist hierbei, daß selbst größere Mengen von bis zu 50 % an Lipiden in die Grundlage inkorporiert werden können, ohne daß damit galenisch relevante Nachteile hinsichtlich der Verarbeitungsfähigkeit (Granulierung nicht zwingend erforderlich) eintreten.

Weiterhin sind erfindungsgemäß vesikelbildende Lipide vorhanden. Bevorzugt sind z.B. Lecithin aus Ei oder Sojabohnen, Cholesterol, Sitosterol, Sphingosin, Ceramide, Cerebroside und/oder Sphingomyeline oder Mischungen hiervon.

Lipide alleine sind jedoch in elektrolytenthaltenden Badetabletten mit Brausecharakter nicht in der Lage, eine gleichmäßige Verteilung in der Badeflotte zu ermöglichen, so daß der Zusatz geeigneter Tenside bzw. Emulgatoren notwendig ist.

Dieses Ziel wird erfindungsgemäß durch O/W- und W/O-Tenside, insbesondere Kombinationen hiervon, erreicht. Zu bevorzugten O/W-Tensiden in Form anionischer Tenside gehören Natriumlaurylsulfat, Natriumlaurylethersulfat oder Kohlenhydrat-Tenside, wie oder Mischungen hiervon. Zu geeigneten W/O-Tensiden vom Typ der niedrigpolyoxyethylierten Fettalkohole gehören Macrogollaurylether mit 1 bis 4 EO-Einheiten, Macrogol(5)-oleylether, Macrogol(7)-glycerolcocoat, Kokosfettsäurediethanolamid, Mono/Di/Tri-(C₈-C₂₀-alkyltetraglykolether)-o-phosphorsäureester, Polyoxyethylen-glyceroltrioleat oder Mischungen hiervon. Bei W/O/O/W-Kombinationen von Tensiden entstehen rückfettende Tensidkomplexe, die eine gute Verträglichkeit gewährleisten. Solche Kombinationen sind daher bevorzugt. Das Verhältnis von W/O / O/W-Tensid ergibt sich aus dem gewünschten Emulsionsziel.

Weitere geeignete O/W-Tenside sind polyoxyethylierte Fettsäuren oder Fettalkohole, polyoxyethylierte Ester bzw. Partialester des Ethylenglykols, Glycerols, Propylenglykols oder Sorbitans. Die Verwendung einzelner oder mehrerer O/W-Tenside ist ebenfalls bevorzugt. Weitere geeignete W/O-Tenside sind Polyoxyethylensorbitantristearat und Polyoxypropylen-(15)-stearylether.

Die obengenannten zugesetzten Tenside haben mehrere nicht vorhersehbare günstige Eigenschaften auf die resultierende Badeflotte: Sie ermöglichen zum einen eine gleichmäßige Verteilung der inkohärenten Lipidphase in der Badeflotte, was angesichts der relativ geringen Konzentration nicht vorhersehbar war. Zum anderen ermöglichen sie neben der reinigenden Wirkung auch die Ausbildung eines in der Regel feinporigen, hautpflegenden und lipidhaltigen Schaums. Darüberhinaus wird überraschenderweise die Liposomenbildung durch die gleichzeitig vorhandenen Tenside nicht beeinträchtigt.

Mit den erfindungsgemäßen Brausetabletten können insgesamt Badeflotten hergestellt werden, die durch den Brausesatz eine belebende und gleichzeitig durch die LipidNesikel-Kombination hautrückfettende Wirkung haben. Diese Effekte können durch Zusatz von Mineralsalzen und/oder wirksamen Komponenten noch verstärkt werden.

Die während der Herstellung der erfindungsgemäßen Zusammensetzungen erhaltenen Produkte können auch ohne Verpressung vewendet werden.

Bei entsprechend erhöhtem Zusatz an Mineralsalzen (z.B. > 80%) und reduzierter Brausesatzmenge ggf. (z.B. < 10%) bis 0%, können auch Granulate und/oder pulverförmige Produkte erhalten werden, die man bereits als solche (d.h. ohne Tablettierung), d.h. als festen balneologischen Badezusatz z.B. als Badesalz einsetzen kann.

Solche pulver- oder granulatförmigen Badesalzzusätze weisen vorteilhafterweise 0-70 % insbesondere 10-50 % des o.g. Brausesatzes und 20-97 % insbesondere 50-97 % Mineralsalze sowie die für die o.g. Brausetabletten erwähnten weiteren Komponenten, nämlich wirksame Komponenten, Hilfsstoffe, Lipidkomponenten, vesikelbildende Lipide, eins oder mehrere O/W und/oder W/O-Tensid(e) sowie Kolloide der angegebenen Art und Menge auf. Dies gilt auch insbesondere für die entsprechend als bevorzugt genannten Komponenten in den entsprechenden Mengenbereichen.

Gewünschtenfalls kann der feste Badesatz auch weniger Brauseanteil aufweisen, ggf. bis 0%, wobei der Mineralienanteil entsprechend angepaßt wird.

So umfaßt der erfindungsgemäße Badezusatz z.B. in Pulver- oder Granulatform neben dem genannten Brausesatz und/oder Mineralsalzen weiterhin 0-30 %, insbesondere 0-10 % wirksame Komponenten ausgewählt aus ätherischen Ölen, Extrakten, Parfümstoffen, Vitaminen, hautpflegenden und kurativen Substanzen oder deren Mischungen, 0 - 10 % Hilfsstoffe, und ist gekennzeichnet durch
0,1-50 %, insbesondere 1-25 % ein oder mehrere Lipidkomponenten;
0,1-20 %, insbesondere 0,1-5 % vesikelbildende Lipide;
0,1-20 %, insbesondere 0,1-5 % ein oder mehrere O/W und/oder W/O-Tensid(e); sowie
0-10 %, insbesondere 0,1-3 % ein oder mehrere Kolloide mit einer Molekülmasse bis maximal 50 000.

Vorzugsweise besteht der Brausesatz hierbei aus Natrium(bi)-, Calcium-, Ammonium- oder Kalium(bi)carbonat oder deren Mischungen und einer oder mehrerer Säuren, die ausgewählt sind aus Zitronensäure, Weinsäure, Fumarsäure, Adipinsäure; Säureanhydride, ausgewählt aus Zitronensäure-, Bernsteinsäureanhydrid oder deren sauren Salzen, ausgewählt aus Natriumdihydrogenphosphat, Natriumfumarat oder deren Mischungen.

Ferner werden hier vorzugsweise als Hilfsstoffe Stoffe mit adsorbierenden Eigenschaften, ausgewählt aus Cyclodextrin, Dextrin, Stärke und/oder hochdisperser Kieselsäue oder andere Siliciumdioxid-Produkte eingesetzt.

Darüberhinaus können vorzugsweise als Kolloid Eiweißhydrolysate pflanzlichen und/oder tierischen Urspungs in einer Menge von 0-10 % enthalten sein.

Als Mineralsalze eignen sich insbesondere Salze, bestehend aus den Kationen Natrium, Kalium, Magnesium, Calcium, Eisen, Ammonium oder Mangan und den Anionen, Hydrogencarbonat, Carbonat, Chlorid, Fluorid, Sulfat ode Nitrat.

Als Lipidkomponente können vorzugsweise hydriertes Sojaöl, hydriertes Rizinusöl, hydriertes Erdnußöl, Partialester und Ester des Glycerols, des Sorbitols, des Ethylenglykols und des Propylenglykols sowie Mischester einschließlich der Sorbitanester sowie Wachse, Ester höherer Fettalkohole und/oder Fettsäuren oder Mischungen hiervon enthalten sein.

Als vesikelbildendes Lipid können vorzugsweise Phospholipide, Lecithin aus Ei oder Sojabohnen, Cholesterol, Sitosterol, Sphingosin, Ceramide, Cerebroside und/oder Sphingomyeline oder Mischungen hiervon enthalten sein.

Als Tenside werden insbesondere O/W-Tenside ausgewählt aus Natriumlaurylsulfat, Natriumlaurylethersulfat, polyoxyethylierte Fettsäuren oder Fettalkohole, polyoxyethylierte Ester bzw. Partialester des Ethylenglykols, Glycerols, Propylenglykols oder Sorbitans, Kohlenhydrat-Tenside oder Mischungen hiervon, und/oder ein oder mehrer W/O-Tenside, ausgewählt aus niedrigpolyoxyethylierten Fettalkoholen, wie z.B. Macrogollaurylether mit 1 bis 4 EO-Einheiten, Macrogol (5)-oleylether, Macrogol(7)-glycerolcocoat, Kokosfettsäurediethanolamid, Mono/Di/Tri-(alkyltetraglykolether)-o-phosphorsäureester, Polyoxyethylen-glyceroltrioleat verwendet.

Die erfindungsgemäßen Badezusätze in Pulver- ode Granulatform werden hergestellt durch Vermischen der Feststoffkomponenten, ggf. (partial) Granulierung und Vermengen mit den flüssigen Komponenten und nachfolgendes Vermischen.

Da weder die aus dem Brausesatz stammenden Salze noch die Mineralsalze, selbst bei hohem Anteil, der Ausbildung liposomaler Strukturen, wie oben erläutert, entgegenwirken, können die erfindungsgemäßen Zusätze wie erwähnt in Badeflotten (Wasser) verwendet werden, wobei Dispersionen liposomaler oder vesikulärer Strukturen erhalten werden, die zur Hautbehandlung (Reinigung, Pflege, etc.) besonders geeignet sind.

Im folgenden werden die Herstellung der erfindungsgemäßen Badezusätze sowie ihre Eigenschaften beschrieben.

### I. Herstellungsbeispiele:

Die Produkte gemäß den folgenden Beispielen 1-7 wurden hergestellt durch Vermischen der festen Produkte und nach anschließender Vereinigung mit den flüssigen Komonenten direkt verpreßt bzw. pulverisiert oder granuliert. Die Produkte gemäß den Beispielen 1-5 enthalten dabei jeweils einen Brausesatz. Die Produkte gemäß den Beispielen 1-3 können als Badetabletten vorliegen; die Produkte gemäß den Beispielen 6 und 7 enthalten keinen Brausesatz und können als Pulver oder Granulat eingesetzt werden.

| Herstellungsbeispiel 1 (Prozentangaben) | |
|---|---|
| Campher | 0,25 |
| Eucalyptusöl | 0,25 |
| Teebaumöl | 0,50 |
| Alpenkräuterextrakt | 1,00 |
| Citronensäure | 36,70 |
| Natriumbicarbonat | 37,70 |
| Hydr. Sojabohnenöl | 7,00 |
| Sojalecithin | 1,00 |
| Natriumlaurylsulfat | 1,00 |
| Laureth 2 | 1,00 |
| Weizenproteinhydrolysat | 1,00 |
| Maisstärke | 0,50 |
| Kaliumbicarbonat | 1,00 |
| Magnesiumcarbonat | 1,00 |
| Calciumcarbonat | 5,00 |
| Natriumchlorid | 2,00 |
| Natriumsulfat | 2,00 |
| Patentblau | 0,10 |
| Parfümöl | 1,00 |

| Herstellungsbeispiel 2 (Prozentangaben) | |
|---|---|
| Vitamin E-acetat | 1,00 |
| Vitamin C | 5,00 |
| Calciumpantothenat | 0,50 |
| Weidenrindenextakt | 0,50 |
| Weinsäure | 42,20 |
| Kaliumbicarbonat | 12,20 |
| Natriumcarbonat | 30,00 |
| Hydr. Erdnußöl | 1,00 |
| Phospholipide | 2,00 |
| Natriumlaurylethersulfat | 1,00 |
| Laureth 4 | 1,50 |
| Collagenhydrolysat | 2,00 |
| Cochenillerot | 0,10 |
| Parfümöl | 1,00 |

| Herstellungsbeispiel 3 (Prozentangaben) | |
|---|---|
| Methylnicotinat | 0,50 |
| Hydroxyethylsalicylat | 0,20 |
| Grüner Tee-Extrakt | 1,00 |
| Citronensäure | 30,00 |
| Fumarsäure | 14,00 |
| Ammoniumcarbonat | 9,20 |
| Natriumbicarbonat | 35,00 |
| Hydr. Rizinusöl | 5,00 |
| Cyclodextrin | 2,00 |
| Sitosterol | 1,00 |
| Kokosfettsäurediethanolamid | 0,50 |
| Natriumlaurylsulfat | 1,00 |
| Polyvinylpyrrolidon | 0,50 |
| Chinolingelb | 0,05 |
| Parfümöl | 0,05 |

| Herstellungsbeispiel 4 (Prozentangaben) | |
|---|---|
| Eucalyptusöl | 0,50 |
| Campher | 0,50 |
| Citronensäure | 19,00 |
| Natriumbicarbonat | 20,00 |
| Hydr. Erdnußöl | 2,00 |
| Cydodextrin | 0,50 |
| Sojalecithin | 1,00 |
| Natriumlaurylsulfat | 1.00 |
| Laureth 4 | 0,50 |
| Collagenhydrolysat | 2,00 |
| Kartoffelstärke | 0,50 |
| Meersalz | 49,40 |
| Natriumsulfat | 2,00 |
| Patentblau | 0,10 |
| Parfümöl | 1,00 |

| Herstellungsbeispiel 5 (Prozentangaben) | |
|---|---|
| Rosmarinöl | 1,50 |
| Vitamin E-succinat | 1,00 |
| Vitamin C | 3,00 |
| Calciumpantothenat | 0,50 |
| Weinsäure | 3,00 |
| Fumarsäure | 4,00 |
| Natriumcarbonat | 8,00 |
| Hydr. Rizinusöl | 1,00 |
| Sitosterol | 2,00 |
| Natriumlaurylethersulfat | 1,00 |
| Laureth 2 | 1,50 |
| Glycerolmonostearat | 0,50 |
| Collagenhydrolysat | 2,00 |
| Cochenillerot | 0,05 |
| Parfümöl | 1,00 |
| Kaliumhydrogencarbonat | 1,00 |
| Steinsalz | 67,95 |
| Natriumsulfat | 1,00 |

| Herstellungsbeispiel 6 (Prozentangaben) | |
|---|---|
| Methylnicotinat | 0,50 |
| Hydroxyethylsalicylat | 0,20 |
| Steinsalz | 45,00 |
| Meersalz | 47,25 |
| Hydr. Rizinusöl | 2,00 |
| Cyclodextrin | 2,00 |
| Sitosterol | 1,00 |
| Kokosfettsäurediethanolamid | 0,50 |
| Natriumlaurylsulfat | 1,00 |
| Chinolingelb | 0,05 |
| Parfümöl | 0,50 |

| Herstellungsbeispiel 7 (Prozentangaben) | |
|---|---|
| Teebaumöl | 0,50 |
| Alpenkräuterextrakt | 1,00 |
| Hydr. Sojabohnenöl | 8,00 |
| Sojalecithin | 1,00 |
| Macrogol-20-stearylether | 1,00 |
| Laureth 2 | 1,00 |
| Weizenproteinhydrolysat | 1,00 |
| Dextrin | 0,50 |
| Magnesiumoxid | 1,00 |
| Natriumchlorid | 2,00 |
| Natriumsulfat | 2,00 |
| Patentblau | 0,10 |
| Parfümöl | 1,00 |
| Pfannensiedesalz | 79,90 |

### II. Nachweis der Verteilung der Lipidphase und von Liposomen mit erfindungsgemäßen Brausetabletten qemäß Herstellunqsbeispiel 1.

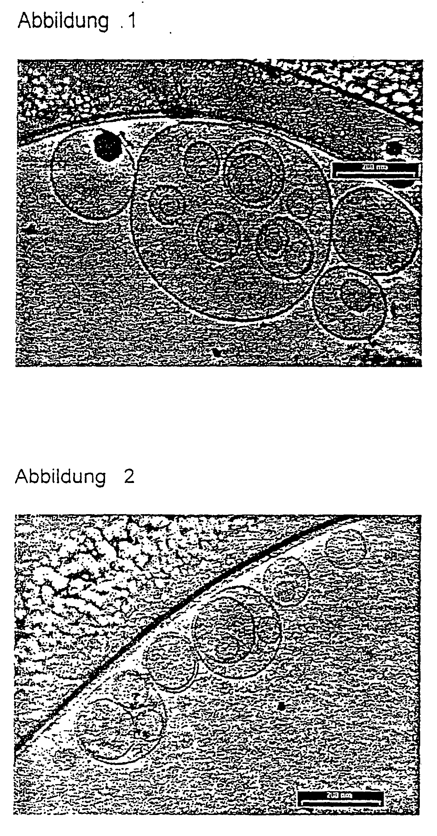

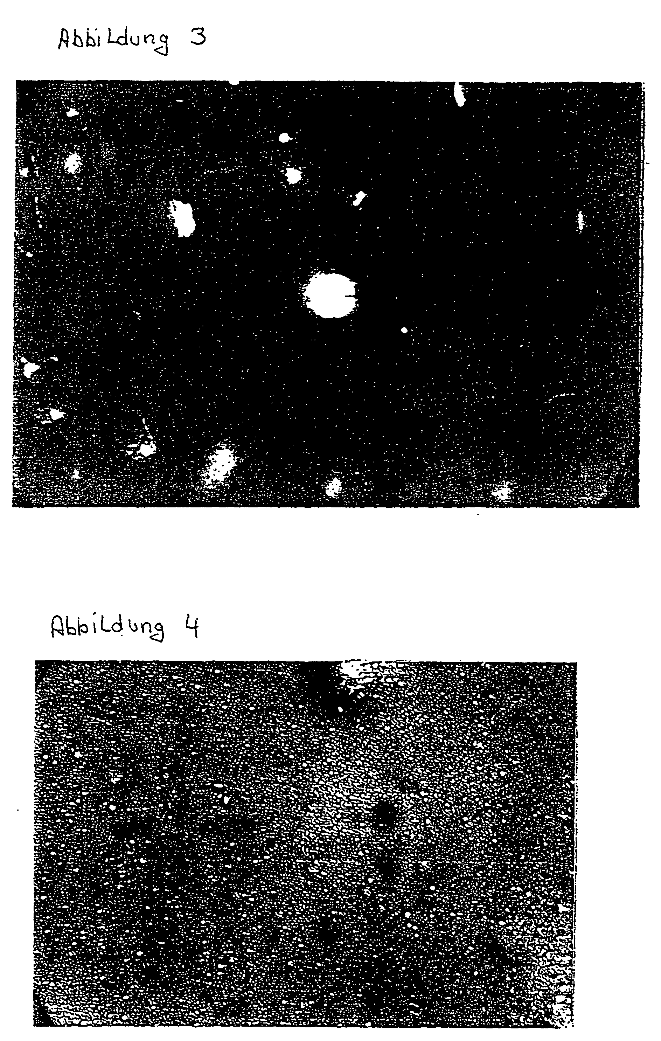

### 1) Abbildungen 1 und 3 (Elektronenmikroskopische Aufnahmen)

In den Abb. 1 und 2 ist die Situation in einer mit dem Produkt gemäß Beispiel 1 hergestellten Badeflotte elektronenmikroskopisch dargestellt.

Hierin sind deutlich (vgl. Kreise) multilamellare (Abb. 1) bzw. bilamellare (Abb. 2) vesikuläre Strukturen zu erkennen.

### 2) Abbildungen 3 und 4 (Polarisationsmikroskopische Aufnahmen)

Da in den elektronenmikroskopischen Aufnahmen Lipidtröpfchen (d.h. die Verteilung der Fettphase) aufgrund ihrer Größe nicht darstellbar sind, wurden polarisationsmikroskopische Aufnahmen der Situation in einer mit dem Produkt gemäß Beispiel 1 hergestellten Badeflotte gemacht.

In Abb. 3 sieht man wiederum deutlich (weiße größere Kreise) die lamellaren Strukturen, in Abb. 4 die gleichmäßige Verteilung der Fettröpfchen.

## Patentansprüche

1. Balneologischer Badezusatz in Form einer Brausetablette, umfassend 10-97 % einer Carbonat-/Bicarbonat-Säure-Mischung, 0 - 30 % wirksame Komponenten ausgewählt aus ätherischen Ölen, Extrakten, Parfümstoffen, Vitaminen, hautpflegenden und kurativen Substanzen oder deren Mischungen, 0 - 10 % Hilfsstoffe, **dadurch gekennzeichnet, daß** die Brausetablette weiterhin
0, 1 -50 % einer oder mehrerer Lipidkomponenten;
0,1-20 % vesikelbildende Lipide;
0, 1 -20 % eines oder mehrerer O/W und/oder W/O-Tensid(e)s;
0-10 % eines oder mehrerer Kolloide mit einer Molekülmasse bis maximal 50.000; sowie
0-80% Mineralsalze
aufweist.

2. Fester balneologischer Badezusatz in pulvriger oder granulierter Form umfassend 0-70% einer Carbonat-/Bicarbonat-Säure-Mischung, 0- 30% wirksame Komponenten ausgewählt aus ätherischen Ölen, Extrakten, Parfümstoffen, Vitaminen, hautpflegenden und kurativen Substanzen oder deren Mischungen, 0- 10% Hilfsstoffe, **dadurch gekennzeichnet, daß** der Zusatz weiterhin
0,1.50 % einer oder mehrerer Lipidkomponenten,
0,1-20 % vesikelbildende Lipide;
0, 1 -20 % eines oder mehrerer O/W und/oder W/O-Tensid(e)s;
0-1 0 % eines oder mehrerer Kolloide mit einer Molekülmasse bis maximal 50 000; sowie
20-97 % Mineralsalze
aufweist.

3. Badezusatz nach einer der Ansprüche 1-2, **dadurch gekennzeichnet, daß** er Natrium(bi)-, Calcium-, Ammonium- oder Kalium(bi)carbonat oder deren Mischungen und eine oder mehrere Säuren, ausgewählt aus Zitronensäure, Weinsäure, Fumarsäure, Adipinsäure; Säureanhydride, ausgewählt aus Zitronensäure-, Bernsteinsäureanhydrid oder deren saure Salze, ausgewählt aus Natriumdihydrogenphosphat, Natriumfumarat oder deren Mischungen als (Bi)Carbonat/Säure-Mischung enthält.

4. Badezusatz nach einem der Ansprüche 1-3, **dadurch gekennzeichnet, daß** als Hilfsstoffe Stoffe mit adsorbierenden Eigenschaften, ausgewählt aus Cyclodextrin, Dextrin, Stärke und/oder hochdisperser Kieselsäure, oder andere Siliciumdioxid-Produkte enthalten sind.

5. Badezusatz nach einem der Ansprüche 1-4, **dadurch gekennzeichnet, daß** er als Kolloid Eiweißhydrolysate pflanzlichen und/oder tierischen Ursprungs in einer Menge von 0- 10% enthält.

6. Badezusatz nach einem der Ansprüche 1- 5, **dadurch gekennzeichnet, daß** als Mineralsalze Salze, bestehend aus den Kationen Natrium, Kalium, Magnesium, Calcium, Eisen, Ammonium oder Mangan und den Anionen, Hydrogencarbonat, Carbonat, Chlorid, Fluorid, Sulfat oder Nitrat, eingesetzt werden.

7. Badezusatz nach einem der Ansprüche 1 oder 3- 6, **dadurch gekennzeichnet, daß** er eine Gesamtmasse von 10- 300 g aufweist.

8. Badezusatz nach einem der Ansprüche 1- 7, **dadurch gekennzeichnet, daß** als Lipidkomponente hydriertes Sojaöl, hydriertes Rizinusöl, hydriertes Erdnußöl, Partialester und Ester des Glycerols, des Sorbitois, des Ethylenglykols und des Propytenglykols sowie Mischester einschließlich der Sorbitanester sowie Wachse, Ester höherer Fettalkohole und/oder Fettsäuren oder Mischungen hiervon enthalten sind.

9. Badezusatz nach einem der Ansprüche 1- 8, **dadurch gekennzeichnet, daß** als vesikelbildendes Lipid Phospholipide, Lecithin aus Ei oder Sojabohnen, Cholesterol, Sitosterol, Sphingosin, Ceramide, Cerebroside und/oder Sphingomyeline oder Mischungen hiervon enthalten sind.

10. Badezusatz nach einem der Ansprüche 1- 9, **dadurch gekennzeichnet, daß** als O/W Tenside Natriumlauryisulfat, Natriumlaurylethersulfat, polyoxyethylierte Fettsäuren oder Fettalkohole, polyoxyethylierte Ester bzw. Partialester des Ethylenglykols, Glycerols, Propylenglykols oder Sorbitans, Kohlenhydrat-Tenside oder Mischungen hiervon, und/oder ein oder mehrere W/O-Tenside, ausgewählt aus niedrigpolyoxyethylierten Fettalkoholen, wie z.B. Macrogollaurylether mit 1 bis 4 EO-Einheiten, Macrogol (5)oleylether, Macrogol(7)-glycerolcocoat, Kokosfettsäurediethanolamid, Mono/Di/Tri(alkyltetraglykolether)-o-phosphorsäureester, Polyoxyethylen-glyceroltrioleat enthalten sind.

11. Verfahren zur Herstellung eines baineologischen Badezusatzes gemäß einem der Ansprüche 1- 10, **dadurch gekennzeichnet, daß** man die einzelnen Feststoffkomponenten vermischt, (partial-)granuliert und mit flüssigen Komponenten in geeigneter Weise vereinigt und ggf. verpreßt, oder nach dem Vermischen und Vereinigen sämtlicher Komponenten ohne Granulation direkt verpreßt oder ohne Verpressung verwendet.

12. Verwendung von Badezusätzen gemäß einem der Ansrüche 1 -11 zur Herstellung einer Dispersion von vesikulären Strukturen oder Liposomen in Wasser zur Hautbehandlung.

## Claims

1. Balneological bath additive in the form of an effervescent tablet, containing 10 to 97% of a carbonate/bicarbonate-acid mixture, 0 to 30% active components selected from ethereal oils, extracts, scents, vitamins, skin-care and curative substances or mixtures thereof, 0 to 10% auxiliary substances, **characterised in that** the effervescent tablet contains in addition
0.1 to 50% of one or more lipid components;
0.1 to 20% vesicle-forming lipids;
0.1 to 20 % of one or more O/W and/or W/O surfactant (s) ;
0 to 10% of one or more colloids having a molecular mass of at most 50,000;
as well as
0 to 80% mineral salts.

2. Solid balneological bath additive in pulverulent or granulated form, containing 0 to 70% of a carbonate/bicarbonate-acid mixture, 0 to 30% active components selected from ethereal oils, extracts, scents, vitamins, skin-care and curative substances or mixtures thereof, 0 to 10% auxiliary substances, **characterised in that** the additive contains in addition
0.1 to 50% of one or more lipid components;
0.1 Co 20% vesicle-forming lipids;
0.1 to 20 % of one or more O/W and/or W/O surfactant (s) ;
0 to 10% of one or more colloids having a molecular mass of at most 50,000;
as well as
20 to 97% mineral salts.

3. Bath additive according to one of claims 1 and 2, **characterised in that** it contains sodium (bi)carbonate, calcium (bi)carbonate, ammonium (bi)carbonate or potassium (bi)carbonate or mixtures thereof and one or more acids, selected from citric acid, tartaric acid, fumaric acid, adipic acid; acid anhydrides, selected from citric anhydride, succinic anhydride, or their acid salts, selected from sodium dihydrogen phosphate, sodium fumarate or mixtures thereof in the form of a (bi)carbonate-acid mixture.

4. Bath additive according to one of claims 1 to 3, **characterised in that** the auxiliary substances contained are substances having adsorbent properties, selected from cyclodextrin, dextrin, starch and/or highly disperse silica, or other forms of silicon dioxide.

5. Bath additive according to one of claims 1 to 4, **characterised in that** it contains as colloid protein hydrolysates of vegetable and/or animal origin in a quantity of 0 to 10%.

6. Bath additive according to one of claims 1 to 5, **characterised in that** the mineral salts used are salts consisting of the cations sodium, potassium, magnesium, calcium, iron, ammonium or manganese and of the anions hydrogen carbonate, carbonate, chloride, fluoride, sulfate or nitrate.

7. Bath additive according to one of claims 1 or 3 to 6, **characterised in that** it has a total mass of 10 to 300 g.

8. Bath additive according to one of claims 1 to 7, **characterised in that** the lipid components contained are hydrogenated soya oil, hydrogenated castor oil, hydrogenated peanut oil, partial esters and esters of glycerol, of sorbitol, of ethylene glycol and of propylene glycol as well as mixed esters, including the sorbitan esters, as well as waxes, esters of higher fatty alcohols and/or fatty acids or mixtures of these.

9. Bath additive according to one of claims 1 to 8, **characterised in that** the vesicle-forming lipids contained are phospholipids, lecithin from egg or from soya beans, cholesterol, sitosterol, sphingosine, ceramides, cerebrosides and/or sphingomyelins or mixtures of these.

10. Bath additive according to one of claims 1 to 9, **characterised in that** the O/W surfactants contained are sodium lauryl sulfate, sodium lauryl ether sulfate, polyoxyethylated fatty acids or fatty alcohols, polyoxyethylated esters or partial esters of ethylene glycol, of glycerol, of propylene glycol or of sorbitan, carbohydrate surfactants or mixtures of these, and/or one or more W/O surfactants, selected from low-polyoxyethylated fatty alcohols such as, for example, macrogol lauryl ethers having 1 to 4 EO units, macrogol (5) oleyl ether, macrogol (7) glycerol cocoate, coconut fatty acid diethanolamide, mono/di/tri(alkyltetraglycol ether) o-phosphoric esters, polyoxyethylene glycerol trioleate.

11. Process for preparing a balneological bath additive according to one of claims 1 to 10, **characterised in that** the individual solid components are mixed together, (partially) granulated and combined in a suitable manner with liquid components and optionally moulded, or, after all the components have been mixed together and combined, they are moulded directly without granulation or are used without having been moulded.

12. Use of bath additives according to one of claims 1 to 11 for preparing a dispersion of vesicular structures or liposomes in water for treatment of the skin.

## Revendications

1. Additif pour le bain sous la forme de comprimé effervescent pour la balnéothérapie, comprenant 10-97% d'un mélange carbonate/bicarbonate acide, 0-30% de composants actifs choisis parmi des huiles essentielles, des extraits, des parfums, des vitamines, des substances de soin de la peau et curatives ou leurs mélanges, 0-10% d'adjuvants, **caractérisé en ce que** le comprimé effervescent comprend en outre
0,1-50% d'un ou plusieurs composants lipidiques ;
0,1-20% de lipides formant des vésicules ;
0,1-20% d'un ou plusieurs tensioactif(s) O/W et/ou W/O ;
0-10% d'un ou plusieurs colloïdes avec une masse moléculaire jusqu'au maximum 50 000 ; ainsi que
0-80% de sels minéraux.

2. Additif solide pour le bain pour la balnéothérapie sous forme de poudre ou de granulés comprenant 0-70% d'un mélange carbonate/bicarbonate acide, 0-30% de composants actifs choisis parmi des huiles essentielles, des extraits, des parfums, des vitamines, des substances de soin de la peau et curatives ou leurs mélanges, 0-10% d'adjuvants, **caractérisé en ce que** l'additif comprend en outre
0,1-50% d'un ou plusieurs composants lipidiques ;
0,1-20% de lipides formant des vésicules ;
0,1-20% d'un ou plusieurs tensioactif(s) O/W et/ou W/O ;
0-10% d'un ou plusieurs colloïdes avec une masse moléculaire jusqu'au maximum 50 000 ; ainsi que
20-97% de sels minéraux.

3. Additif pour le bain selon l'une des revendications 1-2, **caractérisé en ce qu'**il contient du (bi)carbonate de sodium, du carbonate de calcium, du carbonate d'ammonium ou du (bi)carbonate de potassium ou leurs mélanges et un ou plusieurs acides choisis parmi l'acide citrique, l'acide tartrique, l'acide fumarique, l'acide adipique; anhydrides choisi parmi l'anhydride citrique, l'anhydride succinique ou leurs sels acides, choisis parmi le phosphate dihydrogéné de sodium, le fumarate de sodium ou leurs mélanges comme mélange (bi)carbonate/acide.

4. Additif pour le bain selon l'une des revendications 1-3, **caractérisé en ce que** sont contenus comme adjuvants des produits avec des propriétés adsorbantes, choisis parmi la cyclodextrine, la dextrine, l'amidon et/ou l'acide fluorosilicique fortement dispersé, ou d'autres produits de type dioxyde de silicium.

5. Additif pour le bain selon l'une des revendications 1-4, **caractérisé en ce qu'**il contient comme colloïde des hydrolysats protéiniques d'origine végétale et/ou animale en une quantité de 0-10%.

6. Additif pour le bain selon l'une des revendications 1-5, **caractérisé en ce qu'**on utilise comme sels minéraux des sels constitués de cations sodium, potassium, magnésium, calcium, fer, ammonium ou manganèse et des anions carbonate hydrogéné, carbonate, chlorure, fluorure, sulfate ou nitrate.

7. Additif pour le bain selon l'une des revendications 1 ou 3-6, **caractérisé en ce qu'**il présente une masse totale de 10-300 g.

8. Additif pour le bain selon l'une des revendications 1-7, **caractérisé en ce que** sont contenus comme composants lipidiques l'huile de soja hydrogénée, l'huile de ricin hydrogénée, l'huile d'arachide hydrogénée, l'ester partiel et l'ester de glycérol, de sorbitol, d'éthylèneglycol et de propylèneglycol ainsi que leurs esters mixtes, y compris l'ester de sorbitan ainsi que des cires, et des esters d'alcools gras et/ou d'acides gras supérieurs ou leurs mélanges.

9. Additif pour le bain selon l'une des revendications 1-8, **caractérisé en ce que** sont contenus comme lipides formant des vésicules des phospholipides, la lécithine d'oeuf ou de soja, le cholestérol, le sitostérol, la sphingosine, le céramide, le cérébroside et/ou la sphingomyéline ou leurs mélanges.

10. Additif pour le bain selon l'une des revendications 1-9, **caractérisé en ce que** sont contenus comme tensioactifs O/W le laurylsulfate de sodium, le lauryléthersulfate de sodium, les acides gras ou les alcools gras polyoxyéthylénés respectivement les esters partiels d'éthylèneglycol, de glycérol, de propylèneglycol ou de sorbitan, des tensioactifs hydrates de carbone ou leurs mélanges, et/ou un ou plusieurs tensioactifs W/O choisis parmi les alcools gras faiblement polyoxyéthylénés, comme par exemple les éthers lauryliques de macrogol avec 1 à 4 unités EO, l'éther oléylique de macrogol (5), le cocoate de macrogol (7) glycérol, le diéthanolamide d'acide gras de coprah, l'ester d'acide mono/di/tri(alkyltétraglycoléther)-o-phosphorique, le trioléate de polyéthylèneglycol.

11. Procédé de préparation d'un additif pour le bain pour la balnéothérapie selon l'une des revendications 1-10, **caractérisé en ce qu'**on mélange des composants solides individuels, on les granule (partiellement) et on les combine d'une manière appropriée avec les composants liquides et éventuellement on les comprime, ou après le mélange et la combinaison on comprime tous les composants directement sans granulation ou on les utilise sans compression.

12. Utilisation d'additifs de bain selon l'une des revendications 1-11 pour la préparation d'une dispersion de structures vésiculaires ou de liposomes dans l'eau pour le traitement de la peau.
